# EUROPEAN PATENT APPLICATION

(11) **EP 1 130 107 A1**
(43) Date of publication of application: **05.09.2001**
(21) Application number: 01100694.7
(22) Date of filing: 12.01.2001
(51) Int. Cl.: C12P 13/04, C12P 13/22, C12P 41/00

(54) **Method of producing optically active N-methylamino acids**

(30) Priority: 13.01.2000 JP 2000004822
(71) Applicant: KANEKA CORPORATION, Osaka-shi, Osaka 530-8288 (JP)
(72) Inventor: Tsuda, Satoru, Akashi-shi, Hyogo 673-0877 (JP); Kato, Takahisa, Kobe-shi, Hyogo 655-0872 (JP); Yasohara, Yoshihiko, Himeji-shi, Hyogo 670-0942 (JP); Hasegawa, Junzo, Akashi-shi, Hyogo 674-0057 (JP)
(74) Representative: Gille Hrabal Struck Neidlein Prop Roos

(57) **Abstract**

A method of producing an optically active N-methylamino acid of the general formula (2): 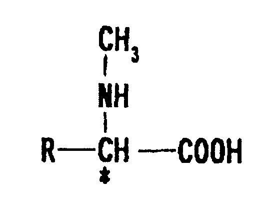 wherein R represents a hydrogen atom or an alkyl, alkenyl, alkynyl, cycloalkyl, aryl or heterocycle residue group, which may optionally have one or more substituents,
which comprises reacting an α-keto acid compound of the general formula (1): 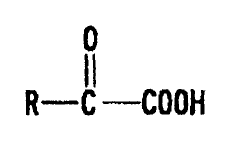 wherein R is as defined above,
with methylamine
using a microorganism cell and/or processed matter thereof, said microorganism being able to convert the carbonyl group of said α-keto acid compound stereoselectively to a methylamino group.

## Description

### FIELD OF THE INVENTION

The present invention relates to a method of producing an optically active N-methylamino acid. An optically active N-methylamino acid is useful as a starting material or an intermediate for the synthesis of medicinals, among others.

### PRIOR ART

As the method of producing an optically active N-methylamino acid, there is known in the art a method comprising using an optically active amino acid as the starting material and methylating the amino group in the manner of chemical synthesis using methyl iodide and sodium hydride [Benoiton et al.: Can. J. Chem., 55 (5), 916 (1977)]. This method has problems, however; it requires the use of methyl iodide and sodium hydride in large amounts and, furthermore, for introducing only one methyl group, the procedure comprising protection and deprotection of the amino group is necessary. On the other hand, as a method utilizing an enzymatic reaction, there is reported a method comprising causing 3-methylaspartase to act on a mixture of fumaric acid and methylamine to give N-methylaspartic acid [Gulzaret et al.: J. Chem. Soc., Perkin Trans. 1, 5, 649 (1997)]. This method, however, requires the use of the enzyme in large amounts and, further requires several days for the reaction, hence the industrial realization thereof is difficult.

### SUMMARY OF THE INVENTION

The present invention provides an efficient method of producing an optically active N-methylamino acid by utilizing the catalytic action of a microorganism.

As a result of intensive investigations made to develop an efficient method of producing an optically active N-methylamino acid, the present inventors discovered certain microorganisms capable of acting on an α-keto acid with methylamine and forming an optically active N-methylamino acid. The discovery of such microorganisms, which has not yet been reported, has led to completion of the present invention.

Thus, the present invention provides a method of producing an optically active N-methylamino acid of the general formula (2): 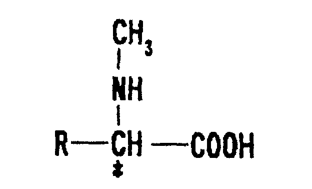 wherein, R represents a hydrogen atom or an alkyl, alkenyl, alkynyl, cycloalkyl, aryl or heterocycle residue group, which may optionally have one or more substituents,
which comprises reacting an α-keto acid compound of the general formula (1) : 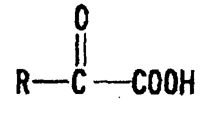 with methylamine
   using a microorganism cell and/or a processed matter thereof,
said microorganism being able to convert the carbonyl group of said α-keto acid compound stereoselectively to a methylamino group.

Further, this invention is also related to a method of producing an optically active N-methylamino acid of the general formula (4) : 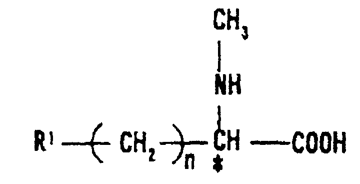 wherein n represents an integer of 0 to 2 and R¹ represents a cycloalkyl, aryl or heterocycle residue group, which may optionally have one or more substituents,
which comprises reacting an α-keto acid compound of the general formula (3): 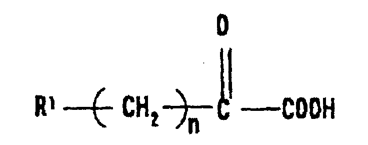 wherein n and R¹ are as defined above,
   with methylamine
   using a microorganism cell and/or processed matter thereof,
said microorganism being able to convert the carbonyl group of said α-keto acid compound stereoselectively to a methylamino group.

### DETAILED DESCRIPTION OF THE INVENTION

In the following, the invention is described in detail. The α-keto acid compound to be used as the substrate in the practice of the invention is represented by the general formula (1) : 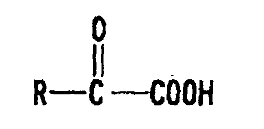 wherein R represents a hydrogen atom or an alkyl, alkenyl, alkynyl, cycloalkyl, aryl or heterocycle residue group, which may optionally have one or more substituents,
and is preferably represented by the general formula (3): 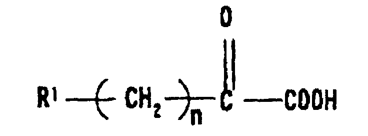 wherein n represents an integer of 0 to 2 and R¹ represents a cycloalkyl, aryl or heterocycle residue group, which may optionally have one or more substituents.

The alkyl group represented by R in the above general formula (1) includes methyl, ethyl, n-propyl, isopropyl, n-butyl, sec-butyl, isobutyl, tert-butyl, n-pentyl, isopentyl, tert-pentyl, heptyl, octyl, nonyl, decyl, etc. and preferred is a C₁-C₅ alkyl group. The alkenyl group includes vinyl, 1-propenyl, 2-propenyl, 1-butenyl, 2-butenyl, 3-butenyl, pentenyl, hexenyl and the like and preferred is a C₂-C₅ alkenyl group. The alkynyl group includes ethynyl, 1-propynyl, 2-propynyl, 1-butynyl, 2-butynyl, 3-butynyl, pentynyl, hexynyl and the like and preferred is a C₂-C₅ alkynyl group. These groups may have one or more substituents. The substituents include halogen atoms and hydroxy, alkoxy, thiol, methylthio, amino, nitro, nitrile, guanidino, carbamoyl and other groups.

The cycloalkyl group represented by R in the general formula (1) and R¹ in the general formula (3) includes cyclopropyl, cyclobutyl, cyclopentyl, cyclohexyl, cycloheptyl and cyclooctyl, for instance, and preferred is a C₄-C₆ cycloalkyl group. The aryl group includes phenyl, naphthyl and the like, and the heterocycle of the heterocycle residue group includes heterocycles containing 1 to 4 hetero atoms selected from among oxygen, sulfur and nitrogen atoms and containing a total of 5 to 10 carbon atoms, such as the furan, dihydrofuran, tetrahydrofuran, pyran, dihydropyran, tetrahydropyran, benzofuran, chromene, thiophene, benzothiophene, pyrrole, pyrroline, pyrrolidine, imidazole, imidazoline, imidazolidine, pyrazole, pyrazoline, triazole, tetrazole, pyridine, piperidine, pyrazolidine, pyrazine, piperazine, pyrimidine, pyridazine, indolidine, indole, isoindole, quinoline, phthalazine, naphthyridine, oxazole, thiazole and morpholine rings. These cycloalkyl, aryl or heterocycle residue groups may be substituted, and the substituents include halogen atoms as well as hydroxy, alkoxy, amino, nitro, nitrile, carboxyl and like groups. More preferred as the α-keto acid compound to be used in the practice of the invention are those compounds in which, referring to the above general formula (3), n is 1 and R¹ is phenyl which may optionally be substituted, more particularly phenyl, 4-chlorophenyl, 4-fluorophenyl or 4-hydroxyphenyl.

Another substrate to be used according to the invention is methylamine. This may be used in the form of an aqueous solution and also in the form of a salt such as hydrochloride.

The microorganism to be used in the practice of the invention may be any microorganism capable of converting the carbonyl group of α-keto acid compounds stereoselectively to a methylamino group and such a microorganism can be screened out in the following manner.

A liquid medium (pH 7) containing 10 g of glucose, 6.5 g of diammonium hydrogen phosphate, 1 g of dipotassium hydrogen phosphate, 0.8 g of magnesium sulfate heptahydrate, 60 mg of zinc sulfate heptahydrate, 90 mg of iron sulfate heptahydrate, 5 mg of copper sulfate pentahydrate, 10 mg of manganese sulfate tetrahydrate, 100 mg of sodium chloride per liter was distributed in 4-ml portions into test tubes, then sterilized and seeded with a microorganism to be tested, and shake culture is carried out at 30 °C for 1 to 2 days. Cells are harvested by centrifugation, washed with physiological saline and suspended in 1 ml of 0.1 M Tris-HCl buffer (pH 8.0) containing 2% of glucose, 1% of sodium phenylpyruvate and 3% of methylamine hydrochloride and shake cultured at 30 °C for 24 hours. After the reaction, the supernatant is analyzed by thin layer chromatography (thin layer: Merck silica gel plate; eluent: n-butanol/acetic acid/water = 4/1/1; color reagent: ninhydrin) to thereby check for the formation or no formation of N-methylphenylalanine.

Examples of the microorganism include microorganisms belonging to the genus *Arthrobacter, Rhodococcus* or *Tsukamurella* and, more specifically, *Arthrobacter histidinolovorans* KNK491 (accession number FERM BP-6955), *Rhodococcus opacus* KNK271 (accession number FERM BP-6956), *Rhodococcus opacus* KNK272 (accession number FERM BP-6957) and *Tsukamurella paurometabola* IFO 12160. Among these microorganisms, the strain IFO 12160 is a known strain, which is readily available from the Institute for Fermentation, Osaka. The other specific microorganisms have been newly isolated and identified from soil by the present inventors and deposited with the Ministry of International Trade and Industry National Institute of Life Science and Human Technology. Their bacteriological characteristics are as shown below.

**Table 1**

| Bacteriological characteristics | | | |
|---|---|---|---|
| | Strain KNK271 | Strain KNK272 | Strain KNK491 |
| Cell morphology | Rods (extensively branched) 0.8 × 3^{~}5 *µ* m | Rods (extensively branched) 1 × 3^{~}5 *µ* m | Rods 0.8 x 1^{~}1.5 µ m |
| Gram stain | Positive | Positive | Positive |
| Spore | - | - | - |
| Motility | - | - | - |
| Colony form | Circular, entire margin smooth, convex, slightly glossy, pale yellow | Circular, entire margin smooth, convex, slightly glossy, pale yellow | Circular, entire margin smooth, slightly convex, glossy, cream-colored |
| Catalase | + | + | + |
| Oxidase | - | - | - |
| O/F test | - | - | - |
| Nitrate reduction | + | + | - |
| Pyrazidamidase | - | - | + |
| Pyrrolidonylallylamidase | - | - | + |
| Alkaline phosphatase | - | - | + |
| β -Glucuronidase | - | - | + |
| β -Galactosidase | - | - | + |
| α-Glucosidase | + | + | + |
| N-Acetyl-β-glucosamidase | - | - | - |
| Esculin (glucosidase) | - | - | + |
| Urease | - | - | - |
| Gelatin liquefaction | - | - | + |
| Carbohydrate fermentation | | | |
| Glucose | - | - | - |
| Ribose | - | - | - |
| Xylose | - | - | - |
| Mannitol | - | - | - |
| Maltose | - | - | - |
| Lactose | - | - | - |
| Sucrose | - | - | - |
| Glycogen | - | - | - |

In cultivating these microorganisms, those media containing nutrients assimilable by these microorganisms can generally be used without any particular restriction. Particularly when glucose is used as the carbon source and an ammonium salt as the nitrogen source, a culture fluid rich in the desired activity can favorably be obtained. In carrying out the cultivation, the addition of an N-methylamino acid compound, such as N-methylphenylalanine, is preferred since a culture fluid high in the desired activity can then be obtained. The N-methylamino acid compound may be in D form, L form or DL form and the addition amount may be not less than 0.01% but preferably is 0.05 to 0.1%.

The cultivation can be carried out under routine conditions, thus at a pH of 4 to 9, preferably 6 to 8, and a temperature of 20 to 40 °C, preferably 25 to 35 °C, aerobically for 1 to 3 days.

The thus-obtained culture fluid may be used as such or microbial cells isolated from the culture fluid may be used. Even a processed matter of the cells, for example as obtained by treatment of the cells with acetone, lyophilization or enzymatic or physical disruption of the cells may be used. It is also possible to extract, from such microbial cells or processed cells, a crude or purified enzyme fraction capable of converting α-keto acid compounds with methylamine to optically active N-methylamino acids by stereoselective methylamination and use the extract. Furthermore, it is possible to use the thus-obtained cells, processed cells, enzyme fraction or the like in the form immobilized on a support. Thus, in the present specification, the term "microorganism cells and/or a processed matter thereof" is used to include, within the meaning thereof, all the above-mentioned microorganism cells, processed matters of microorganism cells, enzyme fraction, and immobilized forms thereof.

The reaction is carried out at a temperature within the range of 10 to 50 °C, preferably 20 to 40 °C, at a pH within the range of 6 to 11, preferably 7 to 10. As for the substrate concentrations during the reaction, that of the α-keto acid compound may be within the range of 0.1 to 2%, preferably 0.1 to 1%, and that of methylamine in the range of 1 to 20 equivalents, preferably 5 to 10 equivalents. It is preferred that the reaction is carried out under conditions of shaking or stirring.

The addition of 0.5 to 10% of such an energy source as glucose or glycerol to the reaction mixture is preferred since better results can then be obtained. The reaction can be promoted by the addition of a coenzyme such as reduced-form nicotinamide adenine dinucleotide (NADH) or reduced-form nicotinamide adeninde dinucleotide phosphate (NADPH) in lieu of such an energy source as mentioned above. These reduced-form coenzymes may be added to the reaction mixture singly or caused to coexist therein together with an enzyme and a substrate therefor for reducing oxidized nicotinamide adenine dinucleotide (NAD+) or oxidized-form nicotinamide adenine dinucleotide phosphate (NADP+) to the reduced form to thereby regenerate the corresponding reduced-form coenzyme. Thus, for example, glucose dehydrogenase may be used as the coenzyme-reducing enzyme and glucose as the substrate therefor, or formate dehydrogenase may be used as the coenzyme-reducing enzyme and formic acid as the substrate therefor.

The optically active N-methylamino acids obtained by the above reaction can be isolated and purified by conventional means, for example by extracting the reaction mixture, either as it is or after separation of cells, with a solvent such as n-butanol and concentrating the extract, followed by crystallization or column chromatography.

The invention makes it possible to produce an optically active N-methylamino acid, which is useful as a starting material or an intermediate for the synthesis of a medicinal, among others, with good efficiency.

### EXAMPLE

The following examples illustrate the invention in further detail.

### Example 1

A liquid medium (pH 7.0) comprising, per liter thereof, 10 g of glucose, 6.5 g of diammonium hydrogen phosphate, 1 g of dipotassium hydrogen phosphate, 0.4 g of magnesium sulfate heptahydrate, 30 mg of zinc sulfate heptahydrate, 45 mg of iron sulfate heptahydrate, 2.5 mg of copper sulfate pentahydrate, 5 mg of manganese sulfate tetrahydrate, 50 mg of sodium chloride and 1 g of N-methyl-L-phenylalanine was distributed in 5-ml portions into large-sized test tubes and sterilized with steam at 121°C for 20 minutes. The medium in each tube was aseptically inoculated with one loopful of one of the microorganisms shown in Table 2, and shake culture was carried out at 30 °C for 24 hours. After incubation, each 4-ml culture fluid was centrifuged, the cells collected were washed once with physiological saline and suspended in 1 ml of 100 mM Tris-HCl buffer (pH 8.0) containing 1% of sodium phenylpyruvate, 3.3% of methylamine hydrochloride and 2% of glucose, the suspension was placed in a test tube, and shake culture was conducted at 30 °C for 24 hours to thereby allow the reaction to proceed. After the reaction, the supernatant was analyzed under the HPLC conditions (1) shown below to determine the yield of N-methylphenylalanine. The configuration and optical purity of the product were determined by converting the product to N-BOC (tert-butoxycarbonyl)-N-methylphenylalanine by tert-butoxycarbonylating the methylamino group in the routine manner, followed by analysis under the HPLC conditions (2) given below.

### [HPLC conditions]

(1) Column: GL Science Zorbax BP-CN
   Eluent: 10 mM phosphate buffer (pH 6.5)/methanol = 99/1
   Flow rate: 1 ml/min
   Column temperature: room temperature
   Detection: UV 210 nm.
(2) Column: Daicel Chemical Industries' Chiralcel OD
   Eluent: Hexane/isopropanol (98/2) solution containing 0.05% trifluoroacetic acid
   Flow rate: 1 ml/min
   Column temperature: room temperature
   Detection: UV 210 nm.

The data thus obtained on the yield, optical purity and absolute configuration of the product N-methylphenylalanine are summarized in Table 2.

**Table 2**

| Microorganism | Yield (mg/ml) | Optical purity (% ee) | Absolute configuration |
|---|---|---|---|
| *R. opacus* KNK271 | 7.3 | 98 | S |
| *R*. *opacus* KNK272 | 7.1 | 98 | S |
| *A histidinolovorans* KNK491 | 6.5 | 98 | S |
| *T*. *paurometabola* IFO 12160 | 4.8 | 95 | S |

### Example 2

*R. opacus* KNK271 was cultivated in the same manner as in Example 1. The reaction was carried out in the same manner as in Example 1 except that the α-keto acid compound shown in Table 3 was used in lieu of sodium phenylpyruvate.

**Table 3**

| α-keto acid | Charge (mg/ml) | Product | Yield (mg/ml) | Optical purity (% ee) | Absolute configuration |
|---|---|---|---|---|---|
| (4-Hydroxyphenyl)-pyruvic acid | 9.0 | N-Methyityrosine | 6.5 | 98 | S |
| (4-Chlorophenyl)-pyruvic acid | 4.0 | N-Methyl-(4-chlorophenyl)-alanine | 3.2 | 97 | S |
| (4-Fluorophenyl)-pyruvic acid | 3.6 | N-Methyl-(4-fluorophenyl)-alanine | 1.5 | 97 | S |

### Example 3

The medium described in Example 1 (50 ml) was placed in a 500-ml Sakaguchi flask, sterilized, and inoculated with 0.5 ml of the culture fluid containing the strain KNK271 as obtained by the cultivation method described in Example 1, and cultivation was carried out at 30 °C for 20 hours. After cultivation, cells were harvested by centrifugation and washed twice with physiological saline. The cells obtained were suspended in 15 ml of 0.1 M phosphate buffer (pH 7.0) containing 5 mM 2-mercaptoethanol and disrupted in a bead beater using 0.5 mm glass beads. Cell fragments were removed by centrifugation and the supernatant was dialyzed against the same phosphate buffer as mentioned above to give 8 ml of a cell-free extract.

A 50-ml three-necked flask was charged with 23.5 ml of 0.1 M Tris-HCl buffer (pH 8), 60 mg of sodium phenylpyruvate, 200 mg of methylamine hydrochloride, 4.2 mg of reduced form nicotinamide adenine dinucleotide (NADH), 70 units of glucose dehydrogenase (trademark: GLUCDH "Amano" II, product of Amano Pharmaceutical), 2 g of glucose and 6.5 ml of the above cell-free extract, and the reaction was allowed to proceed at 30 °C (reaction volume 30 ml). The reaction was carried out with stirring while adjusting the pH to 8.0 using a 1 N aqueous solution of sodium hydroxide. Portions of the reaction mixture were analyzed at intervals by HPLC and, each time the substrate sodium phenylpyruvate was found to have been exhausted, 60 mg thereof was added and the reaction was continued. While repeating this procedure, the reaction was conducted for 24 hours. After completion of the reaction, the yield of N-methylphenylalanie was found to be 350 mg, the conversion from phenylpyruvic acid to be 80.8% and the optical purity to be (S) 98% ee.

## Claims

1. A method of producing an optically active N-methylamino acid of the general formula (2): 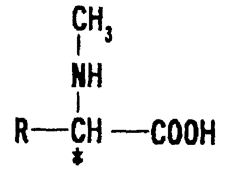 wherein R represents a hydrogen atom or an alkyl, alkenyl, alkynyl, cycloalkyl, aryl or heterocycle residue group, which may optionally have one or more substituents,
which comprises reacting an α-keto acid compound of the general formula (1) : 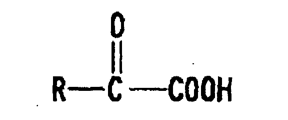 wherein R is as defined above,
with methylamine
using a microorganism cell and/or processed matter thereof,
said microorganism being able to convert the carbonyl group of said α-keto acid compound stereoselectively to a methylamino group.

2. A method of producing an optically active N-methylamino acid of the general formula (4): 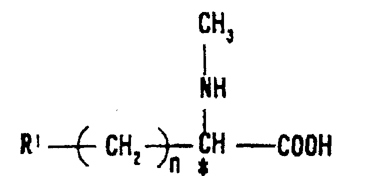 wherein n represents an integer of 0 to 2 and R¹ represents a cycloalkyl, aryl or heterocycle residue group, which may optionally have one or more substituents,
which comprises reacting an α-keto acid compound of the general formula (3): 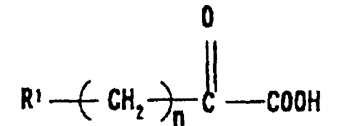 wherein n and R¹ are as defined above,
with methylamine
using a microorganism cell and/or processed matter thereof,
said microorganism being able to convert the carbonyl group of said α-keto acid compound stereoselectively to a methylamino group.

3. The method according to Claim 2,
wherein R¹ is a substituted or unsubstituted phenyl, naphthyl, imidazole ring or indole ring group.

4. The method according to Claim 2,
wherein R¹ is phenyl, 4-chlorophenyl, 4-fluorophenyl or 4-hydroxyphenyl.

5. The method according to any of Claims 1 to 4,
wherein the microorganism belongs to the genus *Arthrobacter, Tsukamurella* or *Rhodococcus.*

6. The method according to any of Claims 1 to 4,
wherein the microorganism is *Arthrobacter histidinolovorans, Rhodococcus opacus* or *Tsukamurella paurometabola.*

7. The method according to any of Claims 1 to 4,
wherein the microorganism is *Arthrobacter histidinolovorans* KNK491 (accession number FERM BP-6955), *Rhodococcus opacus* KNK271 (accession number FERM BP-6956), *Rhodococcus opacus* KNK272 (accession number FERM BP-6957) or *Tsukamurella paurometabola* IFO 12160.
